# EUROPEAN PATENT APPLICATION

(11) **EP 1 351 182 A2**
(43) Date of publication of application: **08.10.2003**
(21) Application number: 03000576.3
(22) Date of filing: 13.01.2003
(51) Int. Cl.: G06F 19/00

(54) **Doctor-evaluation-data providing method and apparatus, and doctor-pay determining method and apparatus, each for remote diagnosis**

(30) Priority: 05.04.2002 JP 2002104450
(71) Applicant: Colin Corporation, Komaki-shi, Aichi-ken (JP)
(72) Inventor: Oka, Tohru, Aichi-ken (JP); Narimatsu, Kiyoyuki, Aichi-ken (JP)
(74) Representative: Winter, Brandl, Fürniss, Hübner, Röss, Kaiser, Polte Partnerschaft

(57) **Abstract**

A method of providing doctor-evaluation data to a patient so that the patient selects, based on the doctor-evaluation data, one of a plurality of doctors remote from the patient and receives a diagnosis made by the selected doctor via a communication line, the method including the steps of updating a set of information that is related to evaluation of the selected doctor and provides part of the doctor-evaluation data stored by a server, based on an evaluation of the selected doctor that has been sent from a terminal device of the patient who has received the diagnosis made by the selected doctor; and sending, in response to a request signal sent from the terminal device of the patient, the doctor-evaluation data to the terminal device.

## Description

### BACKGROUND OF THE INVENTION

### Field of the Invention

The present invention relates to a telediagnosis system that enables a patient to receive a diagnosis made by a doctor at a place remote from the patient, and particularly to doctor-evaluation-data providing method and apparatus, and doctor-pay determining method and apparatus, each for the telediagnosis system.

### Related Art Statement

There is known a telediagnosis system that includes a patient's terminal device set at a place, such as patient's home, remote from a doctor, and a doctor's terminal device that is operated by the doctor and is connected to the patient's terminal device via a communication line, so that the doctor can make a diagnosis on the patient.

Telediagnosis systems have come into easier use because of the spreading of internets and the development of communication lines. However, it is a key to the spreading of telediagnosis systems to assure that the doctors who are engaged with the systems can offer as good as possible medical services. Meanwhile, since a number of doctors can be engaged with a telediagnosis system, each patient can select an appropriate one of the doctors. However, the patient may not be able to easily judge which one of the doctors is appropriate. Thus, it is another key to the spreading of telediagnosis systems to assure that each patient can easily select an appropriate doctor from a plurality of doctors.

### SUMMARY OF THE INVENTION

It is therefore an object of the present invention to provide doctor-evaluation-data providing method and apparatus that assure, for spreading of telediagnosis systems, that doctors can provide better medical services and patients can select better doctors, and doctor-pay determining method and apparatus that assure, for spreading of telediagnosis systems, that doctors can provide better medical services.

The above object has been achieved by the present invention. According to a first aspect of the present invention, there is provided a method of providing doctor-evaluation data to a patient so that the patient selects, based on the doctor-evaluation data, one of a plurality of doctors remote from the patient and receives a diagnosis made by the selected doctor via a communication line, the method comprising the steps of updating a set of information that is related to evaluation of the selected doctor and provides part of the doctor-evaluation data stored by a server, based on an evaluation of the selected doctor that has been sent from a terminal device of the patient who has received the diagnosis made by the selected doctor; sending, in response to a request signal sent from the terminal device of the patient, the doctor-evaluation data to the terminal device.

According to this aspect of the invention, at the doctor-evaluation-data updating step, the set of information that is related to the evaluation of the doctor and provides the part of the doctor-evaluation data is updated based on the evaluation of the doctor that has been made by the patient who has received the diagnosis made by the doctor and, at the data sending step, the thus updated doctor-evaluation data are sent to the terminal device of the patient. Therefore, the patient can select a good doctor based on the thus received doctor-evaluation data, and the doctor always makes an effort to offer better medical services and thereby raise his or her evaluation.

According to a second aspect of the present invention, there is provided an apparatus for providing doctor-evaluation data, comprising a memory device which stores doctor-evaluation data including a plurality of sets of information related to respective evaluations of a plurality of doctors; a doctor-evaluation-data updating means for updating the set of information that is related to the evaluation of each of the doctors and provides part of the doctor-evaluation data stored by the memory device, based on an evaluation of said each doctor that has been sent from a terminal device of a patient who has received a diagnosis made by said each doctor; a data sending means for sending, in response to a request signal sent from the terminal device of the patient, the doctor-evaluation data to the terminal device.

The present doctor-evaluation-data providing apparatus is preferably used to carry out the doctor-evaluation-data providing method according to the first aspect of the invention.

According to a third aspect of the present invention, there is provided a method of determining a pay to be paid to each of a plurality of doctors who are remote from a plurality of patients and from whom each of the patients selects a home doctor in advance so as to receive a diagnosis made by the selected home doctor via a terminal device of the patient and a terminal device of the home doctor that are connected to a communication line, the method comprising the steps of determining, based on a plurality of first sets of identification information identifying the plurality of patients, respectively, and a plurality of second sets of identification information identifying the respective home doctors selected by the patients, a number of the patients who have selected each of the home doctors; and determining, based on the thus determined number of the patients, a pay to be paid to said each home doctor.

According to this aspect of the invention, as the medical services offered by each doctor improve, and as the number of patients who select the each doctor as their home doctor increases, the pay to be paid to the doctor, determined at the pay determining step, increases. Therefore, each doctor makes an effort to offer better medical services.

According to a fourth aspect of the present invention, there is provided an apparatus for determining a pay to be paid to each of a plurality of doctors who are remote from a plurality of patients and from whom each of the patients selects a home doctor in advance so as to receive a diagnosis made by the selected home doctor via a terminal device of the patient and a terminal device of the home doctor that are connected to a communication line, the apparatus comprising a patient-number determining means for determining, based on a plurality of first sets of identification information identifying the plurality of patients, respectively, and a plurality of second sets of identification information identifying the respective home doctors selected by the patients, a number of the patients who have selected each of the home doctors; and a doctor-pay determining means for determining, based on the number of the patients determined by the patient-number determining means, a pay to be paid to the each home doctor.

The present doctor-pay determining apparatus is preferably used to carry out the doctor-pay determining method according to the third aspect of the invention.

### BRIEF DESCRIPTION OF THE DRAWINGS

The above and optional objects, features, and advantages of the present invention will be better understood by reading the following detailed description of the preferred embodiments of the invention when considered in conjunction with the accompanying drawings, in which:
Fig. 1 is a view for explaining the construction of a telediagnosis system to which the present invention is applied;
Fig. 2 is a view for explaining the construction of a server of the system shown in Fig. 1;
Fig. 3 is a view showing an example of patient data stored by a hard disc of the server shown in Fig. 2;
Fig. 4 is a view showing an example of doctor-evaluation data stored by the hard disc of the server of Fig. 2;
Fig. 5 is a block diagram for explaining essential control functions of a CPU (central processing unit) of the server;
Fig. 6 is a flow chart for explaining a process of determining a home doctor;
Fig. 7 is a view showing an example of an image displayed at Step SA3 of Fig. 6;
Fig. 8 is a flow chart for explaining a process of updating the doctor-evaluation data; and
Fig. 9 is a flow chart for explaining a process, carried out by the server, of determining a pay money that is periodically paid to each doctor by a company which runs the telediagnosis system.

### DETAILED DESCRIPTION OF PREFERRED EMBODIMENTS

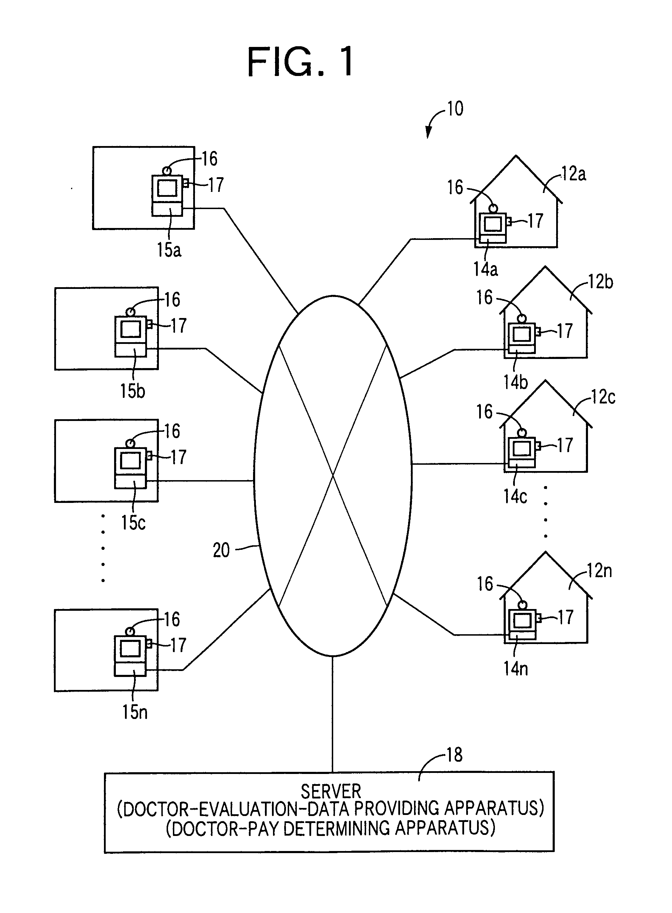

Hereinafter, there will be described a preferred embodiment of the present invention in detail by reference to the drawings. Fig. 1 is a view for explaining the construction of a telediagnosis system 10 to which the present invention is applied.

The telediagnosis system 10 includes a plurality of patient's terminal devices 14a, 14b, 14c, ..., 14n that are provided in respective patient's homes 12a, 12b, 12c, ..., 12n; a plurality of doctor's terminal devices 15a, 15b, 15c, ..., 15n that are operable by respective doctors; and a server 18 that is provided in a company which runs the present telediagnosis system 10 and functions as a doctor-evaluation-data providing apparatus or a doctor-pay determining apparatus. The patient's terminal devices 14, the doctor's terminal devices 15, and the server 18 are connectable to each other via a communication line 20.

Each of the patient's terminal devices 14 and the doctor's terminal devices 15 may be of a stationary type or of a portable type, and may be provided by a common personal computer. Each of the patient's terminal devices 14 and the doctor's terminal devices 15 includes a camera 16 such as a CCD (charge coupled device) camera; a microphone 17; and a speaker, not shown.

The present telediagnosis system 10 is used as follows: Each of the patients selects one of the doctors, and receives a diagnosis made by the selected doctor via the terminal device 14 of the each patient, the communication line 20, and the terminal device 15 of the selected doctor. A patient may receive a diagnosis made by a doctor, either in a direct manner in which the patient and the doctor are simultaneously present in front of their terminal devices 14, 15 that are being connected to each other via the communication line 20, or in an indirect manner in which first the patient sends an electronic mail to the doctor's terminal device 15, then the doctor sends a return electronic mail to the patient's terminal device 14, and so on.

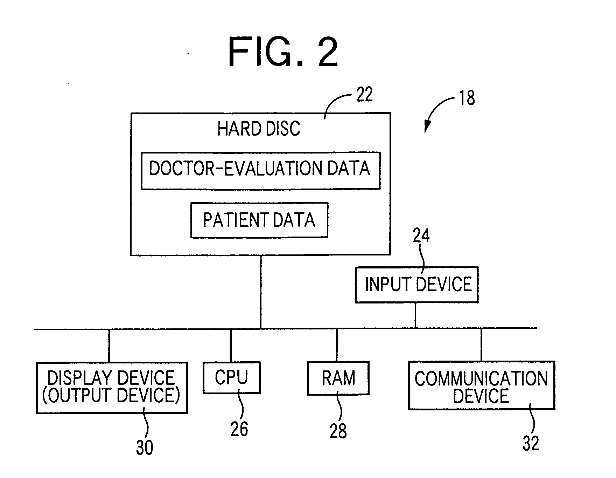

The communication line 20 is provided by, e.g., a wire or wireless internet or LAN. The server 18 is provided by a high-speed and high-capacity electronic computer, and includes a hard disc 22, an input device 24, a CPU (central processing unit) 26, a RAM (random access memory) 28, a display device 30, and a communication device 32, as shown in Fig. 2.

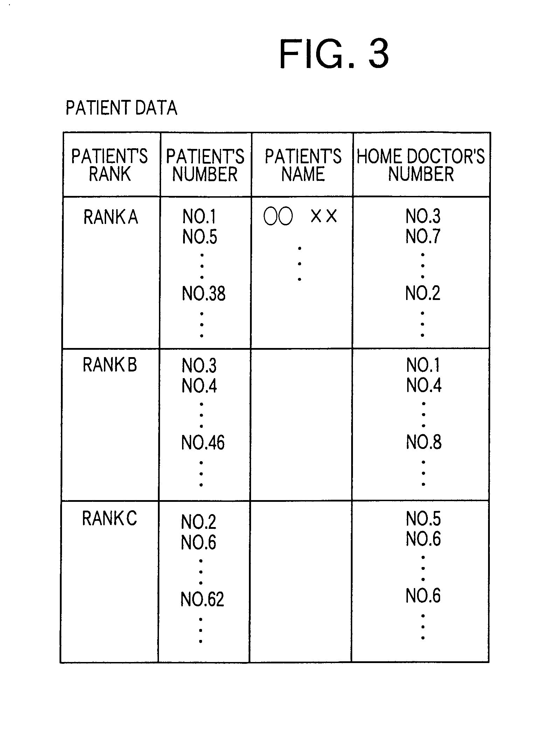

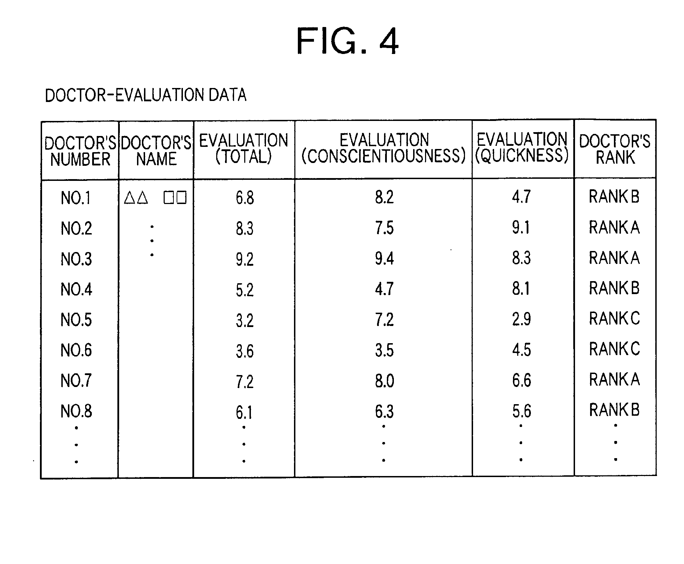

The hard disc 22 functions as a memory device, and stores patient data including a plurality of sets of information related to a plurality of patients who use the present telediagnosis system 10; and doctor-evaluation data including a plurality of sets of information related to respective evaluations of a plurality of doctors who contract with the system-running company. Fig. 3 is a view showing an example of the patient data; and Fig. 4 is a view showing an example of the doctor-evaluation data.

The patient data shown in Fig. 3 include, as the set of information related to each of the patients, a rank, a number, a name, and a home doctor's name for the each patient. The patient's rank is given to the each patient, depending upon a membership fee that is paid by the patient to the system-running company. A patient having a higher rank (Rank "A" is the highest rank) can select a doctor having a higher rank, as his or her home doctor. The patient's number is identification information identifying the each patient; and the home doctor's name for the each patient is identification information identifying the doctor whom the each patient has selected as his or her home doctor.

The doctor-evaluation data shown in Fig. 4 include, as the set of information related to each of the doctors, a number, a name, an evaluation (total), an evaluation (conscientiousness), an evaluation (quickness), and a rank for the each doctor. The three kinds of evaluations are determined based on the respective evaluations of the each doctor that have been made by the patients who have received the respective diagnosis made by the doctor. In the present embodiment, each kind of evaluation is determined as an average of respective points out of ten points that the doctor have got from the respective patients. The doctor's rank is determined based on one or more evaluations determined for the each doctor. In the example shown in Fig. 4, a doctor who has got the evaluation (total) of not less than 7 points is determined as Rank "A"; a doctor who has got the evaluation (total) of not less than 4 points and less than 7 points is determined as Rank "B", and a doctor who has got the evaluation (total) of less than 4 points is determined as Rank "C".

The CPU 26 executes the control programs stored by the hard disc 22, while utilizing the temporary-storage function of the RAM 28, and thereby controls respective operations of the hard disc 22, the display device 30, and the communication device 32. The communication device 32 includes a modem, a terminal adaptor, and a router, and transmits data stored by the hard disc 22, and receives data, via the communication line 20.

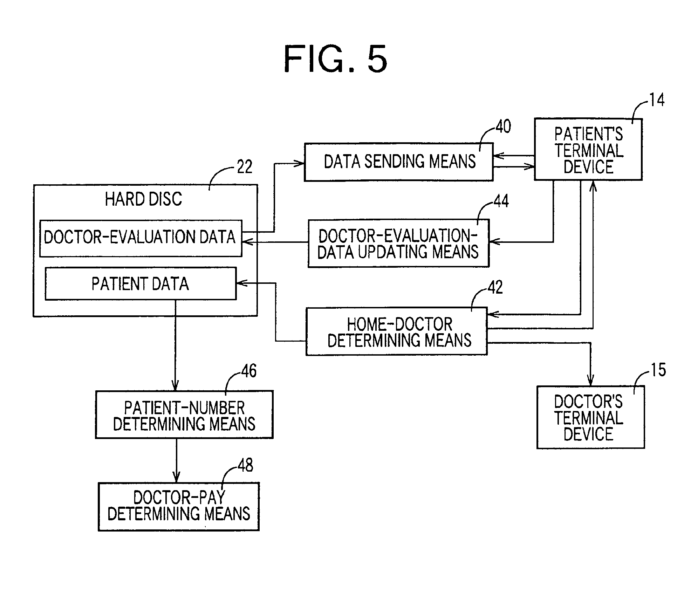

Fig. 5 is a block diagram for explaining essential control functions of the CPU 26 of the server 18. A data sending means 40 sends, in response to a request signal sent thereto from each patient's terminal device 14, the doctor- evaluation data stored by the hard disc 22, to the each patient's terminal device 14.

A home-doctor determining means 42 determines, based on a selection signal sent thereto from each patient's terminal device 14, one of the doctors who has been selected by each patient who operates the each terminal device 14, as the home doctor of the each patient, and stores, as part of the set of information related to the each patient, information representing that the one doctor has been selected as the home doctor of the each patient. In addition, the home-doctor determining means 42 sends, to the each terminal device 14, an electronic mail informing that the one doctor has been selected as the home doctor of the each patient, and additionally sends, to the terminal device 15 of the one doctor, an electronic mail informing that the doctor has been selected as the home doctor of the each patient.

A doctor-evaluation-data updating means 44 updates, based on one or more evaluations of each doctor that has or have been made by one or more patients who have received respective diagnoses made by the each doctor, the set of information that is related to the each doctor and provides part of the doctor-evaluation data stored by the hard disc 22.

A patient-number determining means 46 determines, based on the respective doctor's numbers given to the respective home doctors selected for the respective patients, stored as part of the patient data by the hard disc 22, a number of patients who belong to each of the patient's ranks and have selected each of the doctors as their home doctor.

A doctor-pay determining means 48 determines a pay that is paid by the system-running company to each of the doctors, based on the sum of respective products of the respective numbers of patients belonging to the respective ranks and respective predetermined rates that correspond to the respective ranks and increase with the ranks, that is, the sum of the product of the number of patients belonging to the highest rank and the predetermined highest rate corresponding to the highest rank, the product of the number of patients belonging to the second highest rank and the predetermined second highest rate corresponding to the second highest rank, and so on.

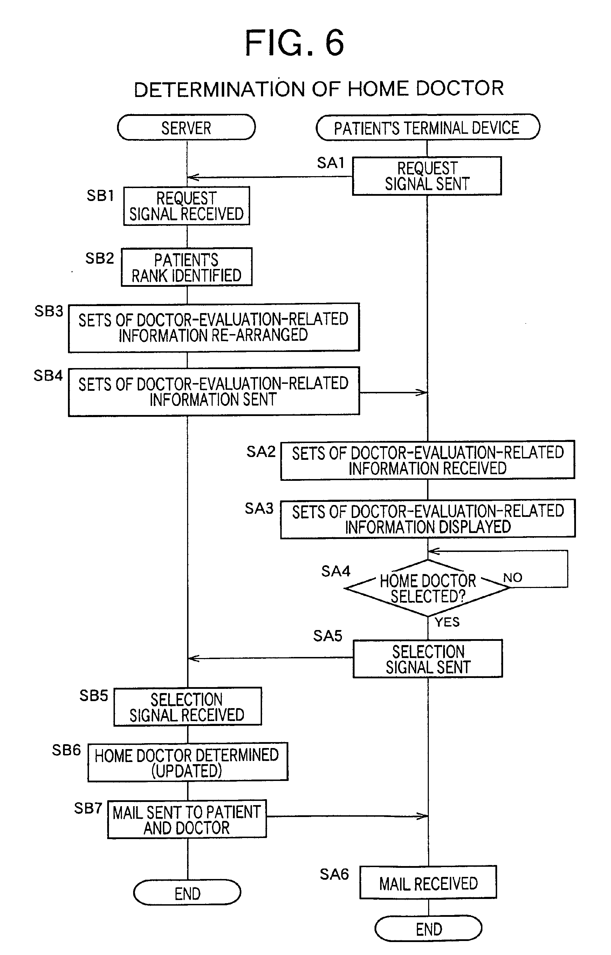

Fig. 6 is a flow chart for explaining a method of determining a home doctor. First, at Step SA1 (hereinafter, "Step(s)" is omitted), each patient's terminal device 14 sends, to the server 18, a request signal requesting the server 18 to send the doctor-evaluation data to the terminal device 14, together with the patient's number identifying the patient who operates the terminal device 14.

Then, SB1 through SB4 corresponding to a data sending step and the data sending means 40 are carried out by the server 18. First, at SB1, the server receives the request signal and the patient's number sent from the patient's terminal device 14. Subsequently, at SB2, the server identifies the patient's rank, based on the thus received patient's number and the patient data stored by the hard disc 22.

Then, at SB3, the server re-arrange the respective sets of information related to the respective evaluations of the doctors, in the order of doctor's ranks, i.e., in the order starting with the doctors having the highest rank and ending with the doctors having the lowest rank. Subsequently, at SB4, the server sends the thus re-arranged sets of information, to the patient's terminal device 14 that had sent the request signal to the server. To the terminal device 14, the server additionally sends a message informing the patient of the doctor's ranks that can be selected by the patient according to the patient' rank.

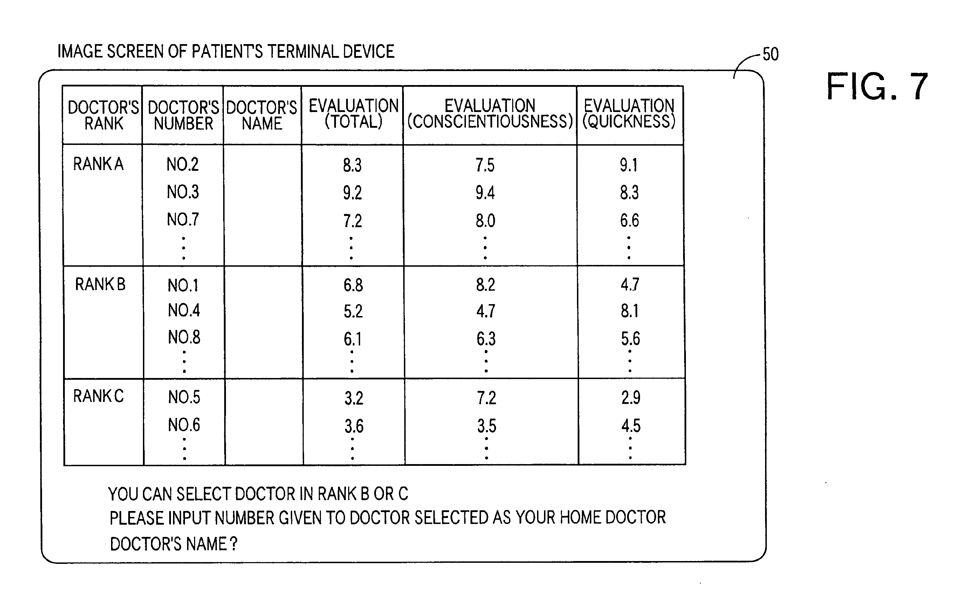

At SA2, the patient' terminal device 14 receives the sets of doctor-evaluation-related information sent from the server 18 and, at SA3, the terminal device 14 displays the sets of doctor-evaluation-related information on an image screen 50 thereof. Fig. 7 shows an example of the sets of doctor-evaluation-related information that are displayed on the display screen 50 at SA3 in the case where the patient's rank identified at SB2 is Rank B. In a lower portion of the image screen 50, a message of "YOU CAN SELECT DOCTOR IN RANK B OR C" is displayed.

The patient selects, based on the sets of doctor-evaluation-related information and the message displayed on the image screen 50 at SA3, one of the doctors as his or her home doctor, and specifies the one doctor by operating a keyboard, not shown, of the patient's terminal device 14. At SA4, the terminal device 14 judges whether the patient has selected his or her home doctor by operating the keyboard. SA4 is repeated until a positive judgment is made. If a positive judgment is made at SA4, the control goes to SA5 to send a selection signal representing the doctor's number given to the doctor selected as the home doctor and the patient's number given to the patient, to the server 18.

After the selection signal is sent at SA5, the server 18 carries out SB5 through SB7 corresponding to the home-doctor determining means 42. First, at SBS, the server receives the selection signal and, subsequently at SB6, the server determines or updates a portion of the set of information related to the patient specified by the selection signal, i.e., a doctor's number given to the doctor selected as the home doctor by the patient. The set of patient-related information is stored as part of the patient data in the hard disc 22. Thus, the server determines or updates the home doctor selected by the patient.

Subsequently, at SB7, the server sends, to the patient's terminal device 14 that had sent the selection signal and the doctor's terminal device 15 operated by the doctor who has been selected by the patient as his or her home doctor, an electronic mail informing what has been determined or updated at SB6. Each doctor contracts with the system-running company for making a diagnosis or giving a medical advise to each patient if the doctor is selected as the patient's home doctor and receives a request from the patient via the communication line 20.

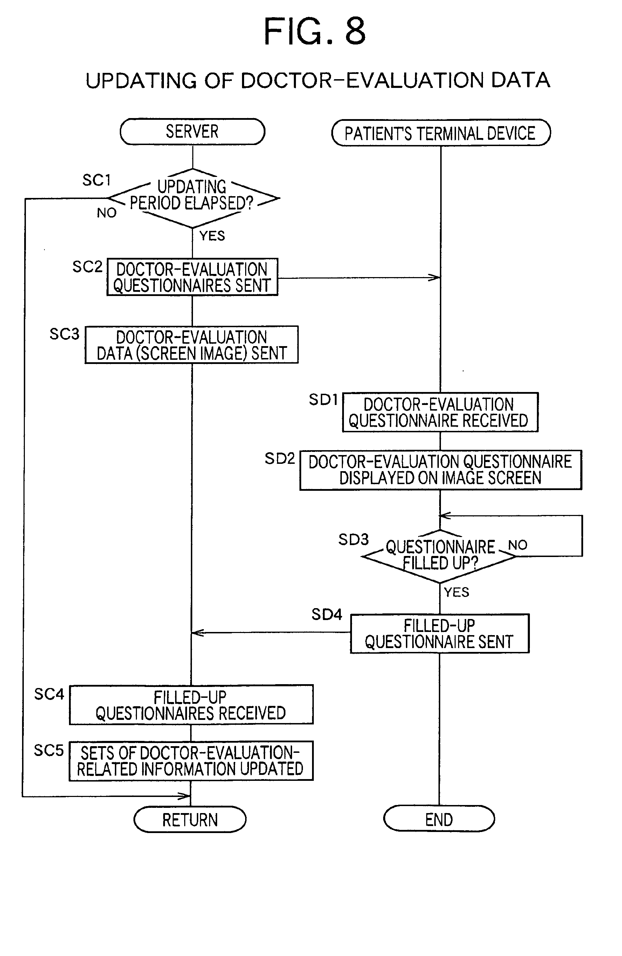

Fig. 8 is a flow chart representing a method of updating the doctor-evaluation data. First, at SC1, the server 18 judges whether a predetermined data-updating period at which the doctor-evaluation data stored in the hard disc 22 are updated has elapsed. The data-updating period may be predetermined at, e.g., one week or one month. If a negative judgment is made at SC1, the server quits this routine at this stage and goes back to the routine shown in Fig. 7. After the routine of Fig. 7 is carried out, the server again carries out SC1.

Meanwhile, if a positive judgment is made at SC1, the control goes to SC2 to send respective doctor-evaluation questionnaires, to all the patient's terminal devices 14. In the case where each set of doctor-evaluation-related information stored in the hard disc 22 of the server 18 includes the three kinds of evaluation, i.e., evaluation (total), evaluation (conscientiousness), and evaluation (quickness), shown in Fig. 4, each doctor-evaluation questionnaire requests each patient to answer the doctor's number (or doctor's name) given to the doctor who had made diagnosis on the patient and make those three kinds of evaluation on the doctor.

After at SC2 the server 18 sends the doctor-evaluation questionnaires, each patient's terminal device 14 receives, at SD1, one of the doctor-evaluation questionnaires. Subsequently, at SD2, the patient's terminal device 14 displays the doctor-evaluation questionnaire on the image screen 50. Then, at SD3, the patient's terminal device 14 judges whether the doctor-evaluation questionnaire has been filled up, that is, all the questions have been answered and additionally an answering-ending action has been done. SD3 is repeated until a positive judgment is made. Meanwhile, if a positive judgment is made at SD3, then the control goes to SD4 to send the filled-up doctor-evaluation questionnaire, that is, doctor evaluation, to the server 18. At SC3, the server sends the periodically updated doctor-evaluation data to each patient's terminal device 14, so that an image representing the updated doctor-evaluation data can be displayed on the screen of the terminal device 14.

When at SD4 the patient's terminal devices 14 send the respective filled-up doctor-evaluation questionnaires, the server 18 receives, at SC4, those filled-up questionnaires. Subsequently, at SC5, the server adds the respective new evaluations of the doctors that are represented by the received questionnaires, to the respective past evaluations of those doctors, calculates respective averages of the thus updated evaluations of the doctors, and stores the thus calculated averages as the respective updated sets of doctor-evaluation-related information. Since the sets of doctor-evaluation-related information are periodically updated, at SC5, based on the respective evaluations made by the patients, the doctor-evaluation data that are sent to the patient's terminal devices 14 at SB4 of Fig. 6 are periodically updated. Thus, SC1 through SC5 correspond to the doctor-evaluation-data updating means 44.

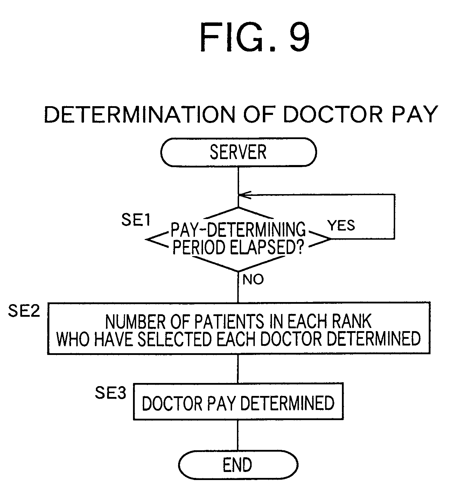

Fig. 9 is a flow chart for explaining an operation of the server 18 for determining a pay that is periodically paid by the system-running company to each of the doctors. First, at SE1, the server 18 judges whether a predetermined pay-determining period has elapsed. This pay-determining period may be predetermined at, e.g., one year.

Subsequently, the control goes to SE2 corresponding to the patient-number determining means 46. At SE2, based on the patient data stored in the hard disc 22, the server determines, for each of the doctors, the number of patients who have selected the each doctor as their home doctor, with respect to each of the patient's ranks. Then, the control goes to SE3 corresponding to the doctor-pay determining means 48. At SE3, the server determines, for each of the doctors, a pay that is paid to the each doctor, such that the pay is equal to a predetermined base pay plus the sum of respective products of the respective determined numbers of patients belonging to the respective patient's ranks and respective predetermined rates that correspond to the respective patient's ranks and increase with the ranks.

It emerges from the foregoing description of the present embodiment that at SC1 through SC5 (the doctor-evaluation-data updating step or the doctor-evaluation-data updating data 44), the server updates, based on the respective evaluations of each of the doctors that are made by the patients who have received the diagnoses made by the each doctor, the corresponding set of doctor-evaluation-related information as part of the doctor-evaluation data, and that at SB1 through SB4 (the data sending step or the data sending means 40), the server sends the thus updated doctor-evaluation data to each one of the patient's terminal devices 14 that has requested the data. Thus, each patient can select, based on the thus sent doctor-evaluation data, an appropriate one of the doctors as his or her home doctor, and each doctor endeavors to provide better medical services so as to raise his or her evaluations.

In addition, in the illustrated embodiment, as the medical services provided by each doctor improve, and as the number of patients who select the each doctor as their home doctor increases, the doctor's pay, determined at SE3 (the pay determining step or the pay determining means 48), increases. Therefore, each doctor endeavors to provide better medical services.

While the present invention has been described in its embodiment by reference to the drawings, it is to be understood that the present invention can otherwise be embodied.

For example, in the above-described embodiment, the doctor-evaluation data sent to each patient's terminal device 14 are used by each patient for selecting, in advance, the patient's home doctor who will make a diagnosis on the patient. That is, in the above embodiment, when the doctor-evaluation data are sent to the patient's terminal device 14, the patient selects, based on the thus sent doctor-evaluation data, the patient's home doctor from the doctors. However, the doctor-evaluation data may be used by each patient for selecting an appropriate doctor each time the patient wishes to obtain a medical diagnosis.

In addition, in the above embodiment, each time the predetermined doctor-evaluation-data updating period elapses, respective doctor-evaluation questionnaires are sent to the patient's terminal devices 14, so that the patient's terminal devices 14 send, periodically at the updating period, the respective doctor evaluations. However, for example, each patient's terminal device 14 may store such a doctor-evaluation sending program that is automatically started when each patient receives a diagnosis made by a doctor, cannot be ended unless the patient inputs his or her evaluation of the doctor, and automatically sends the evaluation of the doctor.

It is to be understood that the present invention may be embodied with various changes or improvements that may occur to a person skilled in the art without departing from the scope and spirit of the present invention.

## Claims

1. A method of providing doctor-evaluation data to a patient so that the patient selects, based on the doctor-evaluation data, one of a plurality of doctors remote from the patient and receives a diagnosis made by the selected doctor via a communication line, the method comprising the steps of:
updating a set of information that is related to evaluation of the selected doctor and provides part of the doctor-evaluation data stored by a server, based on an evaluation of the selected doctor that has been sent from a terminal device of the patient who has received the diagnosis made by the selected doctor, and
sending, in response to a request signal sent from the terminal device of the patient, the doctor-evaluation data to the terminal device.

2. An apparatus (10) for providing doctor-evaluation data, comprising:
a memory device (22) which stores doctor-evaluation data including a plurality of sets of information related to respective evaluations of a plurality of doctors;
a doctor-evaluation-data updating means (44) for updating the set of information that is related to the evaluation of each of the doctors and provides part of the doctor-evaluation data stored by the memory device, based on an evaluation of said each doctor that has been sent from a terminal device (14) of a patient who has received a diagnosis made by said each doctor; and
a data sending means (40) for sending, in response to a request signal sent from the terminal device of the patient, the doctor-evaluation data to the terminal device.

3. A method of determining a pay to be paid to each of a plurality of doctors who are remote from a plurality of patients and from whom each of the patients selects a home doctor in advance so as to receive a diagnosis made by the selected home doctor via a terminal device of the patient and a terminal device of the home doctor that are connected to a communication line, the method comprising the steps of
determining, based on a plurality of first sets of identification information identifying the plurality of patients, respectively, and a plurality of second sets of identification information identifying the respective home doctors selected by the patients, a number of the patients who have selected each of the home doctors; and
determining, based on the thus determined number of the patients, a pay to be paid to said each home doctor.

4. An apparatus (10) for determining a pay to be paid to each of a plurality of doctors who are remote from a plurality of patients and from whom each of the patients selects a home doctor in advance so as to receive a diagnosis made by the selected home doctor via a terminal device (14) of the patient and a terminal device (15) of the home doctor that are connected to a communication line (20), the apparatus comprising:
a patient-number determining means (46) for determining, based on a plurality of first sets of identification information identifying the plurality of patients, respectively, and a plurality of second sets of identification information identifying the respective home doctors selected by the patients, a number of the patients who have selected each of the home doctors; and
a doctor-pay determining means (48) for determining, based on the number of the patients determined by the patient-number determining means, a pay to be paid to said each home doctor.
